# EUROPÄISCHE PATENTSCHRIFT

(11) **EP 3 462 868 B1**
(45) Veröffentlichungstag und Bekanntmachung des Hinweises auf die Patenterteilung: **19.07.2023**
(21) Anmeldenummer: 17723424.2
(22) Anmeldetag: 18.05.2017
(51) Int. Cl.: A01N 31/02, A01N 25/30, A01P 15/00, A61Q 19/10, A61K 8/34, A61Q 5/02, A61K 8/44, A61K 8/46

(54) **MIKROBIOLOGISCH STABILE TENSIDHALTIGE FORMULIERUNG**
MICROBIOLOGICALLY STABLE SURFACTANT-CONTAINING FORMULATION
FORMULE TENSIOACTIVE MICROBIOLOGIQUEMENT STABLE

(30) Priorität: 23.05.2016 EP 16170893
(43) Veröffentlichungstag der Anmeldung: 10.04.2019
(73) Patentinhaber: Evonik Operations GmbH, 45128 Essen (DE)
(72) Erfinder: JÄNICHEN, Jan, 22149 Hamburg (DE); PETERSEN, Wilfried, 22143 Hamburg (DE)
(74) Vertreter: Evonik Patent Association
(86) Internationale Anmeldenummer: PCT/EP2017/061940
(87) Internationale Veröffentlichungsnummer: WO 2017/202677

(56) Entgegenhaltungen:
- EP-A1- 1 287 108
- WO-A1-03/066012
- WO-A1-2017/109423
- CN-A- 101 332 160
- FR-A1- 2 780 283
- JP-A- H11 269 023
- JP-A- H11 279 023
- "Exemptions from the requirement of a tolerance in pesticide formulations", FOCUS ON SURFACTANTS, ELSEVIER ENGINEERING INFORMATION, INC, US, Bd. 2010, Nr. 10, 1. Oktober 2010 (2010-10-01), Seite 4, XP027395510, ISSN: 1351-4210 [gefunden am 2010-10-01]
- Hellerbach Alexandra ET AL: "MRI Phantoms - Are There Alternatives to Agar?", PLoS ONE, vol. 8, no. 8, 5 August 2013 (2013-08-05), page e70343, XP055828514, DOI: 10.1371/journal.pone.0070343

## Beschreibung

### Gebiet der Erfindung

Die Erfindung betrifft eine wässrige Formulierung gemäß dem Oberbegriff des Anspruchs 1.

### Stand der Technik

Konservierungsmittel werden in tensidhaltigen Produkten, insbesondere in kosmetischen Produkten eingesetzt, in denen die Anwesenheit von Wasser und biologisch verwertbaren Materialien wie Fetten, Ölen und oberflächenaktiven Verbindungen eine ideale Matrix für das Wachstum von Bakterien, Hefen und Pilzen bilden.

Die Kontrolle des Wachstums von Mikroorganismen in diesen Produkten ist erforderlich, um sowohl Wirksamkeit und Aussehen der Formulierungen zu erhalten, aber auch um die Sicherheit der Konsumenten zu gewährleisten.

Dem Fachmann ist dabei bekannt, dass Mikroorganismen in der wässrigen Phase gedeihen und die lipophile Umgebung meiden. Zur Bekämpfung von Mikroorganismen in kosmetischen Produkten ist daher nicht nur die antimikrobielle Wirksamkeit eines zu diesem Zweck verwendeten Konservierungssystems von Bedeutung, sondern auch dessen Konzentration in der wässrigen Phase. Viele lipophile Strukturen mit prinzipiell guten antimikrobiellen Eigenschaften eignen sich aufgrund ihres lipophilen Charakters daher nur begrenzt zur Konservierung kosmetischer Produkte.

Vor der Jahrtausendwende wurde der Konservierung insbesondere von kosmetischen und dermatologischen Produkten vielfach wenig Aufmerksamkeit gewidmet. Die Kosten und die Effizienz eines Konservierungsmittels waren für den Hersteller der Produkte ausschlaggebend, und oft wurde mangelnde Produktionshygiene durch den exzessiven Gebrauch von hochwirksamen Konservierungsmitteln kompensiert. Zusätzlich waren die Kriterien zur Bestimmung der mikrobiologischen Stabilität so anspruchsvoll, dass exzessive Gebrauchsmengen von hochaktiven und chemisch wirkenden Konservierungsmitteln wie Formaldehyd/Formaldehyd-Abspaltern und/oder Isothiazolinonen ihren Weg in kosmetische und dermatologische Mischungen fanden. Als Konsequenz aus deren exzessiven Gebrauch wurden beim Konsumenten kosmetischer Produkte eine wachsende Zahl von Reizungen und Sensibilisierungen beobachtet, welche nachweislich auf diese chemisch wirkenden Konservierungsmittel zurückgeführt werden konnten. Als Konsequenz auf diese Befunde wurden viele der traditionellen Konservierungsmittel daher für die Verwendung insbesondere in kosmetischen Formulierungen von Gesetzesseite eingeschränkt oder ihr öffentliches Ansehen hat stark gelitten.

Für den Entwickler tensidhaltiger, insbesondere kosmetischer Produkte, bedeuten die zunehmenden Einschränkungen in der Verwendung von Mitteln zur mikrobiologischen Stabilisierung solcher Produkte eine große Herausforderung. Seiner gesetzlichen Verpflichtung zur Vermarktung ausschließlich mikrobiologisch sicherer Produkte auf der einen Seite stehen Anforderungen des Konsumenten, nur auf ausgewählte traditionelle Konservierungsmittel zurückzugreifen oder am besten ganz auf solche Systeme zu verzichten, auf der anderen Seite gegenüber. Des Weiteren ist ein solcher Entwickler konzeptbedingt vielfach mit Rahmenbedingungen, wie z.B. dem Vermarktungskonzept oder dem pH-Wert der Formulierung, konfrontiert, welche die Verwendung bestimmter Konservierungssysteme entgegenstehen.

Unterliegen tensidhaltige Produkte keinerlei pH-Restriktionen, so können sie gewöhnlich im sauren pH-Bereich zwischen pH = 4,5 - 5,5 formuliert und erfolgreich mit organischen Säuren wie z.B. Benzoesäure oder Sorbinsäure gegen mikrobiellen Befall stabilisiert werden. Erfordert das Produktkonzept jedoch pH-Werte oberhalb dieses Niveaus oder solche, die eher im neutralen Milieu liegen, so verlieren organische Säuren ihre Wirksamkeit. Problemverschärfend kommt bei derartigen pH-Bedingungen hinzu, dass eine Vielzahl pH-unabhängig wirkender Konservierungssysteme, wie z.B. Phenoxyethanol, erfahrungsgemäß in tensidhaltigen Formulierungen nur eine stark eingeschränkte Wirksamkeit aufweisen. Während nämlich in emulsionsbasierten Produkttypen die Verteilung des Konservierungssystems von dessen individueller Löslichkeit in der Öl- bzw. Wasserphase abhängt, gestaltet sich die Situation in tensidbasierten Formulierungen komplexer. Derartige Systeme sollen eine gute Reinigungskraft besitzen und sich bequem in ihrer Viskosität modifizieren lassen, um gebrauchsfertige Produkte herzustellen. Realisiert werden diese Anforderungen vorzugsweise mit Hilfe von anionischen und amphoteren Tensidkomponenten, welche in der Lage sind, oberflächlich geladene micellare Strukturen auszubilden. Deren Packungsdichte wirkt sich auf die Beweglichkeit der Micellen aus und bestimmt damit die rheologischen Eigenschaften der Formulierung. In solchen Micellen jedoch können die genannten pH-unabhängigen Konservierungssysteme wie z.B. Phenoxyethanol eingeschlossen werden. Damit werden sie der wässrigen Phase entzogen und können keinen ausreichenden Schutz gegen potentiell anwesende Mikroorganismen entfalten.

Dies gilt besonders, wie dem Fachmann bekannt, in Tensidsystemen auf Basis von Kombinationen anionischer Tenside wie Laurylsulfaten, Laurylethersulfaten oder Acylglutamaten mit Acylamidoalkylbetainen oder Alkylbetainen.

Dem Entwickler fehlt es konsequenterweise an Alternativen für eine sichere Stabilisierung z.B. annähernd pH-neutraler Tensidformulierungen mit von der Öffentlichkeit akzeptierten Konservierungsmitteln.

Der Erfindung liegt die Aufgabe zu Grunde, die oben beschriebene Lücke effizienter Konservierungssysteme für tensidhaltige Produkte im Allgemeinen und für kosmetische Produkte zur Reinigung von Haut und Haar im Besonderen zu schließen und dem Entwickler solcher Produkte ein zuverlässiges antimikrobiell wirksames System für derartige Produktkonzepte zur Verfügung zu stellen.

### Beschreibung der Erfindung

Die Erfindung löst diese Aufgabe durch den Gegenstand des Hauptanspruchs. Vorteilhafte Ausgestaltungen sind in den Unteransprüchen beschrieben.

Gegenstand der Erfindung ist somit eine wässrige Formulierung enthaltend:
a) mindestens ein anionisches Tensid ausgewählt aus der Gruppe der Alkylsulfate, der Alkylethersulfate und/oder der Acylglutamate,
b) mindestens ein amphoteres Tensid ausgewählt aus der Gruppe der Alkylbetaine und/oder Amidoalkylbetaine,
c) 2-Methyl-1,3-propandiol,
dadurch gekennzeichnet, dass
d) sie einen pH Wert von 6 bis 10 aufweist,
e) zur mikrobiologischen Stabilisierung keine weiteren Konservierungsmittel enthält ausgewählt aus der Gruppe bestehend aus:
e1) im Anhang V der VO (EG) Nr. 1223/2009 genannten, zugelassenen Konservierungsmittel ausgewählt aus Benzoesäure und ihr Natriumsalz und andere Salze der Benzoesäure und Benzoesäureester, Propionsäure und ihre Salze, Salicylsäure und ihre Salze, 2,4-Hexadiensäure und ihre Salze, Formaldehyd und Paraformaldehyd, 2-Hydroxybiphenyl und seine Salze, Pyrithionzink, Anorganische Sulfite und Bisulfite , Chlorobutanol, 4-Hvdroxvbenzoesäure, ihre Salze und Ester, 3-Acetyl-6-methyl-2,4(3H)-pyrandion und seine Salze, Ameisensäure und ihr Natriumsalz, 1,6-Bis(4-amidino-2-bromphenoxy)-n-hexan (Dibromhexamidin) und seine Salze einschließlich Isethionat, Thiomersal, Phenylguecksilber und seine Salze einschließlich Borat, 10-Undecvlensäure und seine Salze, 5-Pvrimidinamin, 1,3-Bis(2-ethylhexyl)hexahydro-5-methyl, 5-Brom-5-nitro-1,3-dioxan, Bronopol, 2,4-Dichlorbenzylalkohol, 1-(4-Chlorphenyl)-3-(3,4-dichlorphenyl)-harnstoff, Chlorkresol, 5-Chloro-2-(2,4-dichlorophenoxv)-phenol, Chloroxylenol, N,N"-Methylenbis[N'-[3-(hydroxymethyl)-2.5-dioxoimidazolidin-4-yl]harnstoff, Poly(methylen), α, ω-Bis[[[(aminoiminomethyl)aminoliminomethyl]amino]-, Dihydrochlorid, 2-Phenoxyethanol, Methenamin, 1-(3-Chloroallyl)-3,5,7-triaza-1-azonia- adamantanchlorid, 1-(4-Chlorphenoxy)1-(1H-imidazol-1-yl)-3,3-dimethyl-2-butanon, 1,3-Bis-(hydroxy-methyl)-5,5-dimethyl-2,4-imidazolidindion, Benzylalkohol, 1-Hydroxy-4-methyl-6-(2,4,4-trimethyl- pentvl)-2-pvridon und sein Monoethanolaminsalz, 2,2'-Methylenbis(6-brom-4-chlorphenol) (Bromchlorophen), 3-Methyl-4-(1-methyläthyl)phenol, Gemisch von 5-Chlor-2-methyl-3(2H)-isothiazolon und 2-Methyl-3(2H)-isothiazolon mit Magnesiumchlorid und Magnesiumnitrat, Chlorophen, 2-Chloracetamid, N,N"-Bis(4-chlorphenyl)-3,12-diimino-2,4,11,13-tetraaza-tetradecandiimidamid, sein Azetat, Gluconat und Hydrochlorid, 3-Phenoxv-1-propanol, Alkyl(C12-22)trimethylammoniumbromid und -chlorid, 4,4-Dimethyl-1,3-oxazolidin, N-Hydroxymethyl-N-[1,3-di(hydroxy-methyl)-2,5-dioxo-imidazolidinyl-4-yl]-TN-hydroxymethyl-harnstoff, Benzolcarboximidamid, 4,4'-(1,6-Hexandivlbis(oxv))bis- und seine Salze darunter Isethionat und þ-Hydroxybenzoat, Glutaraldehyd (1,5-pentandial), 5-Ethyl-3,7-dioxa-1-azabicvclo [3.3.0] octan, 3-(p-Chlorohenoxy)-1,2-prooandiol, Natriumhydroxymethylaminoacetat, Silberchlorid aufgebracht auf Titandioxid, Benzolmethanaminium, N,N-Dimethyl-N-[2-[2-[4-(1,1,3,3,-tetramethylbutyl)phenoxy]ethoxy]ethyl]-, Chlorid, Benzalkoniumchlorid, -bromid und -saccharinat, Phenylmethoxy-Methanol-, 3-lod-2-propinylbutytcarbamat, 2-Methyl-2H-isothiazol-3-on, wobei unter dem Begriff "Salze" die Salze der Kationen Natrium, Kalium, Calcium, Magnesium, Ammonium und Äthanolamine und Salze der Anionen Chlorid, Bromid, Sulfat, Azetat sowie unter dem Begriff "Ester" die Methyl-, Ethyl-, Propyl-, Isopropyl-, Butyl-, Isobutyl- und Phenylester verstanden werden, und
e2) multifunktionalen Additiven ausgewählt aus der Gruppe der linearen oder verzweigten, gesättigten oder ungesättigten aliphatischen oder aromatischen Hydroxamsäuren mit einer Anzahl von 6 - 16 Kohlenstoffatomen und aromatischen Alkoholen der allgemeinen Struktur

Ar-(CH2)n-OH

mit n = 2 - 4
dadurch gekennzeichnet, dass 2-Methyl-1,3-propandiol in einer Konzentration von 0,1 bis 8 Gew.-% enthalten ist,
wobei die folgende Zusammensetzung ausgenommen ist:

| INCI | Gew.-% |
|---|---|
| Aqua/water/eau | 86,2225 |
| Sodium laureth sulfate | 3,99 |
| Coco-betaine | 3,90 |
| Methylpropanediol | 2,00 |
| Sodium chloride | 0,91 |
| Pentylene glycol | 0,55 |
| Disodium edta | 0,50 |
| Fragrance (parfum) | 0,50 |
| Sodium citrate | 0,50 |
| Peg-90 glyceryl isostearate | 0,35 |
| Niacinamide | 0,30 |
| Citric acid | 0,14 |
| Camellia sinensis leaf extract | 0,05 |
| Tambourissa trichophylla leaf extract | 0,05 |
| Laureth-2 | 0,0375 |

Die Erfindung hat erkannt, dass 2-Methyl-1,3-propandiol überraschenderweise in einer beanspruchten Zusammensetzung im mildalkalischen Bereich von pH 6 bis 10 als alleiniges Konservierungsmittel eine hinreichende biozide Wirkung aufweist, ohne dass es weiterer Konservierungsmittel bedarf.

Die erfindungsgemäße wässrige Formulierung ist einphasig, besitzt somit ausschließlich eine Wasserphase. Es handelt sich nicht um eine Emulsion.

Erfindungsgemäß wird somit insbesondere die Anwesenheit weiterer Konservierungsmittel gemäß Merkmal e) ausgeschlossen, vorzugsweise sind keinerlei weitere Konservierungsmittel enthalten. 2-Methyl-1,3-propandiol ist im Stand der Technik zwar bereits als Bestandteil kosmetischer Formulierungen bekannt, jedoch war es unbekannt und überraschend, dass dieser Stoff in der beanspruchten Zusammensetzung als alleiniges Konservierungsmittel hinreichende Wirkung entfaltet.

2-Methyl-1,3-propandiol wird u.a. von der Firma Lyondell unter dem Markennamen MP-Diol^{®} Glycol seit vielen Jahren in verschiedenen Industriezweigen vermarktet. Für eine Verwendung in der kosmetischen Industrie wird es von Lyondell vorwiegend aufgrund seiner lösungsvermittelnden Eigenschaften sowohl für hydrophile als auch lipophile Substanzen sowie zur Hautbefeuchtung beworben. Darüber hinaus werden 2-Methyl-1,3-propandiol (INCI: Methylpropandiol) duftverstärkende Eigenschaften in duftstoffhaltigen Formulierungen zugeschrieben (Quelle: Lyondellbassell, MP-Diol^{®} Glycol, "A Product for the Personal Care Industry", 2011).

Die antimikrobiellen Eigenschaften vom 2-Methyl-1,3-propandiol wurden ebenfalls von Lyondell untersucht. Die minimalen Hemmkonzentrationen (MHK) gegen die getesteten Keime lagen zwischen 0,5 und 50%. Für die in kosmetischen Anwendungen relevanten und in einem klassischen kosmetischen Belastungstest nach Pharm. Eur. 2014, 5.1.3 geprüften Mikroorganismen wurden MHK Werte zwischen 10 und 20% und minimale Abtötungskonzentrationen zwischen 20 und 40% nachgewiesen (Quelle: Lyondell Chemical, "Antimicrobial Screen", 2000). Auf Basis dieser Erkenntnisse wurden dem MP-Diol^{®} Glycol unterstützende oder verstärkende Eigenschaften von Konservierungsmitteln zugeschrieben, eine alleinige Verwendung zur Stabilisierung kosmetischer Produkte jedoch nicht angeregt (Quelle: Lyondell Chemical, "Personal Care Formulation Guide Screen", 1998).

Die verstärkende Wirkung von 2-Methyl-1,3-propandiol auf das Konservierungsmittel Phenoxyethanol wird in JP-A 11-279023 offenbart. Ein vergleichbarer Effekt wurde für Paraben in der JP-A 11-269023 beschrieben.

Die Verwendung von 2-Methyl-1,3-propandiol in Produkten zur Reinigung der Haut wird in verschiedenen Patentschriften beschrieben. EP 1334715 B2 offenbart die Kombination von 2-Methyl-1,3-propandiol mit Ölen zur Herstellung von Reinigungsemulsionen in Gegenwart weiterer Konservierungsmittel.

FR 2780283 A1 beschreibt wässrige Formulierungen, welche keine der in Anhang V der Kosmetikrichtlinie genannten Konservierungssysteme beinhalten und deren mikrobiologische Stabilisierung auf einer Kombination von 2-Methyl-1,3-propandiol und einem Komplexbildner beruht. Die in FR 2780283 A1 genannten Ausführungsbeispiele stellen jedoch Emulsionen dar, enthalten keine der in der vorliegenden Anmeldeschrift genannten Tenside und wurden im schwach sauren Milieu (pH 5) formuliert. Darüber hinaus zeigen die in den Tabellen 3 und 4 wiedergegebenen Ergebnisse mikrobiologischer Untersuchungen, dass 2-Methyl-1,3-propandiol alleine keine ausreichende antimikrobielle Wirkung besitzt und die Anwesenheit des Komplexbildners Na4EDTA zwingend erforderlich ist.

Seit mehreren Jahren bietet die Dr. Straetmans GmbH unter dem Markennamen Dermosoft^{®} OMP ein multifunktionelles Konservierungssystem an, welches 2-Methyl-1,3-propandiol enthält und welches für eine grundsätzliche Verwendung in kosmetischen Formulierungen einschließlich solchen zur Reinigung der Haut und Haaren angeboten wird. Das antimikrobielle Prinzip dieses Blends basiert auf der in EP 0524548 B1 beschriebenen synergistischen Wirkung einer Kombination von 1,2-Diolen und Phenylalkanolen, welche vom 2-Methyl-1,3-propandiol verstärkt wird.

Unter dem Namen Spectrastat^{®} G und Spectrastat^{®} H bietet die Firma Inolex multifunktionelle Konservierungssysteme an, welche 2-Methyl-1,3-propandiol neben weiteren Bestandteilen enthalten und welche auch für tensidhaltige kosmetische Formulierungen im neutralen pH-Bereich angeboten werden. Der Inverkehrbringer dieser Blends führt auf seiner Internetseite (http://inolex.com/PC/Products/Preservation-Systems/Spectrastat-Series/Spectrastat-G bzw. -H) die antimikrobielle Wirkung der angebotenen Blends auf die ebenfalls in den Mischungen vorhandenen Rohstoffe Caprylohydroxamsäure und Glyceryl Caprylate bzw. Ethylhexylglycerin zurück. Hinweise auf eine Wirkung ohne die beiden zusätzlichen Komponenten werden nicht gegeben.

Zusammenfassend kann 2-Methyl-1,3-propandiol gemäß dem vorliegenden Stand der Technik demnach als verbreiteter Rohstoff zur Verwendung in kosmetischen Produkten gelten, dessen unterstützende Funktion auf die antimikrobielle Wirkung von Konservierungssystemen in verschiedenen Produkttypen beschrieben wurde. Die Möglichkeit zur Verwendung von 2-Methyl-1,3-propandiol als alleiniger Bestandteil zur mikrobiologischen Stabilisierung einer nahezu pH neutralen oder mildalkalischen und tensidhaltigen Formulierung ist überraschend und wurde bislang noch nicht beschrieben.

In einer alternativen Ausführungsform enthält die erfindungsgemäße Formulierung als Komponente a) mindestens ein anionisches Tensid ausgewählt aus der Gruppe der Alkylsulfate, und/oder der Acylglutamate,
und als Komponente b) mindestens ein amphoteres Tensid ausgewählt aus der Gruppe der Alkylbetaine und/oder Amidoalkylbetaine.

In einer weiteren alternativen Ausführungsform enthält die erfindungsgemäße Formulierung als Komponente a) mindestens ein anionisches Tensid ausgewählt aus der Gruppe der der Alkylsulfate, der Alkylethersulfate und/oder der Acylglutamate,
und als Komponente b) mindestens ein amphoteres Tensid ausgewählt aus der Gruppe der Alkylbetaine.

Erfindungsgemäße tensidhaltige Formulierungen enthalten als waschaktive Komponenten eine Kombination von anionischen und amphoteren Tensiden. Die anionischen Komponenten sind ausgewählt aus der Gruppe der Alkylsulfate, der Alkylethersulfate oder der Acylglutamate. Der Anteil dieser Tenside liegt üblicherweise bei etwa 0,1 bis 30, vorzugsweise 2 bis 25 und insbesondere 5 bis 20 Gew.-%, wobei sich die Gewichtsprozente auf die Gesamtformulierung beziehen.

Die im Rahmen der vorliegenden Erfindung beschriebenen Alkylsulfate können durch Sulfatierung von einem oder mehrerer Fettalkoholen mit einer Kettenlänge von 8-16 C-Atomen und anschließende Neutralisation gewonnen werden. Sie besitzen demnach die allgemeine Struktur: 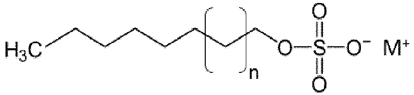
n = 1 - 9
M = z.B. Na+, NH4+

Als Vertreter dieser Gruppe seien exemplarisch aber nicht einschränkend die folgenden Rohstoffe mit ihrer INCI genannt: Sodium Lauryl Sulfate, Ammonium Lauryl Sulfate, Sodium Coco Sulfate.

Werden Fettalkohole mit einer Kettenlänge von 8-16 C-Atomen zunächst mit 1-4 Mol Ethylenoxid umgesetzt und anschließend sulfatiert, so resultieren nach Neutralisation die beanspruchten Alkylethersulfate der allgemeinen Struktur: 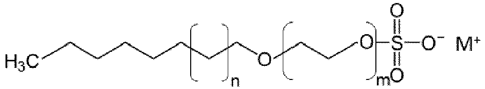
n = 1 - 9
m = 1 - 4
M = z.B. Na+, NH4+

Als Vertreter dieser Gruppe seien exemplarisch aber nicht einschränkend die folgenden Rohstoffe mit ihrer INCI genannt: Sodium Laureth Sulfate, Ammonium Laureth Sulfate, Sodium Coceth Sulfate.

Erfindungsgemäße Acylglutamate können aus Fettsäuren mit einer Kettenlänge von 8-16 C-Atomen und Glutaminsäure hergestellt werden. Nach anschließender Neutralisation besitzen sie die allgemeine Struktur: 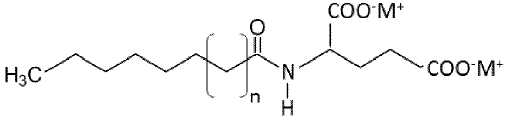
n = 1 - 9
M = z.B. Na+, H+

Als Vertreter dieser Gruppe seien exemplarisch aber nicht einschränkend die folgenden Rohstoffe mit ihrer INCI genannt: Sodium Cocoyl Glutamate, Sodium Lauroyl Glutamate, Disodium Cocoyl Glutamate.

Die anionischen Tenside werden gemäß der vorliegenden Erfindung mit amphoteren Tensiden kombiniert. Der Anteil letzterer Tenside liegt üblicherweise bei etwa 0,1 bis 20, vorzugsweise 1 bis 15 und insbesondere 2 bis 10 Gew.-%, wobei sich die Gewichtsprozente auf die Gesamtformulierung beziehen.

Die erfindungsgemäßen amphoteren Tenside aus der Gruppe der Acylamidoalkylbetaine besitzen die folgende allgemeine Struktur: 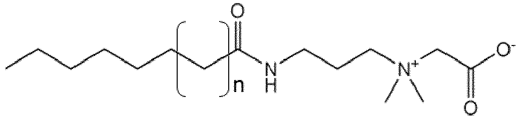 n = 1 - 9

Als Vertreter dieser Gruppe seien exemplarisch aber nicht einschränkend die folgenden Rohstoffe mit ihrer INCI genannt: Cocoamidopropylbetaine, Lauramidopropylbetaine.

Die erfindungsgemäßen amphoteren Tenside aus der Gruppe der Alkylbetaine besitzen die folgende allgemeine Struktur: 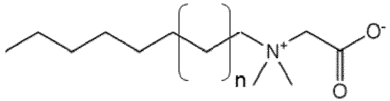 n = 1 - 9

Als Vertreter dieser Gruppe seien exemplarisch aber nicht einschränkend die folgenden Rohstoffe mit ihrer INCI genannt: Coco Betaine, Cetyl Betaine.

Die vorgenannten Kombinationen der waschaktiven Substanzen können erfindungsgemäß mit Hilfe von 2-Methyl-1 ,3-propandiol gegen mikrobiologischen Befall stabilisiert werden. Das 2-Methyl-1 ,3-propandiol kann dabei direkt in die Tensidmischungen eingearbeitet werden, seine Einsatzkonzentration liegt zwischen 0,1 - 8, vorzugsweise 1 - 8 und besonders bevorzugt 2 - 7 Gew.-%, wobei sich die Gewichtsprozente auf die Gesamtformulierung beziehen.

Erfindungsgemäße Formulierungen sind des Weiteren dadurch charakterisiert, dass sie einen pH-Wert von 6 bis 10, vorzugsweise 6 bis 9,5, weiter vorzugsweise 6 bis 9 besitzen.

Der "pH-Wert" im Zusammenhang mit der vorliegenden Erfindung ist definiert als der Wert, bei 25 °C gemessen wird mit einer kalibrierten pH-Elektrode gemäß ISO 4319 (1977).

Erfindungsgemäße Formulierungen weisen keine weiteren Konservierungsmittel ausgewählt aus der folgenden Gruppe aus:
- im Anhang V der VO (EG) Nr. 1223/2009 genannte, zugelassenen Konservierungsmittel, und
- multifunktionale Additiven ausgewählt aus der Gruppe der linearen oder verzweigten, gesättigten oder ungesättigten aliphatischen oder aromatischen Hydroxamsäuren mit einer Anzahl von 6 - 16 Kohlenstoffatomen und aromatischen Alkoholen der allgemeinen Struktur

Ar-(CH2)n-OH

mit n = 2 - 4.

Die VERORDNUNG (EG) Nr. 1223/2009 DES EUROPÄISCHEN PARLAMENTS UND DES RATES vom 30. November 2009 über kosmetische Mittel ist einschließlich des Anhangs V veröffentlicht im Amtsblatt der Europäischen Union, L 342/59 vom 22. Dezember 2009. Der Anhang V dieser Verordnung wird durch Bezugnahme darauf ausdrücklich auch zum Gegenstand und Offenbarungsgehalt der vorliegenden Patentanmeldung gemacht.

Anhang V der VO (EG) Nr. 1223/2009 im Sinne der vorliegenden Erfindung nennt als zugelassene Konservierungsmittel Benzoesäure und ihr Natriumsalz und andere Salze der Benzoesäure und Benzoesäureester, Propionsäure und ihre Salze, Salicylsäure und ihre Salze, 2,4-Hexadiensäure und ihre Salze, Formaldehyd und Paraformaldehyd, 2-Hydroxybiphenyl und seine Salze, Pyrithionzink, Anorganische Sulfite und Bisulfite , Chlorobutanol, 4-Hydroxybenzoesäure, ihre Salze und Ester, 3-Acetyl-6-methyl-2,4(3H)-pyrandion und seine Salze, Ameisensäure und ihr Natriumsalz, 1,6-Bis(4-amidino-2-bromphenoxy)-n-hexan (Dibromhexamidin) und seine Salze einschließlich Isethionat, Thiomersal, Phenylquecksilber und seine Salze einschließlich Borat, 10-Undecylensäure und seine Salze, 5-Pyrimidinamin, 1,3-Bis(2-ethylhexyl)hexahydro-5-methyl, 5-Brom-5-nitro-1,3-dioxan, Bronopol, 2,4-Dichlorbenzylalkohol, 1-(4-Chlorphenyl)-3-(3,4-dichlorphenyl)-harnstoff, Chlorkresol, 5-Chloro-2-(2,4-dichlorophenoxy)-phenol, Chloroxylenol, N,N"-Methylenbis[N'-[3-(hydroxymethyl)-2,5-dioxoimidazolidin-4-yl]harnstoff, Poly(methylen), α, ω-Bis[[[(aminoiminomethyl)amino]iminomethyl]amino]-, Dihydrochlorid, 2-Phenoxyethanol, Methenamin, 1-(3-Chloroallyl)-3,5,7-triaza-1-azonia- adamantanchlorid, 1-(4-Chlorphenoxy)1-(1H-imidazol-1-yl)-3,3-dimethyl-2-butanon, 1,3-Bis-(hydroxy-methyl)-5,5-dimethyl-2,4-imidazolidindion, Benzylalkohol, 1-Hydroxy-4-methyl-6-(2,4,4-trimethyl- pentyl)-2-pyridon und sein Monoethanolaminsalz, 2,2'-Methylenbis(6-brom-4-chlorphenol) (Bromchlorophen), 3-Methyl-4-(1-methyläthyl)phenol, Gemisch von 5-Chlor-2-methyl-3(2H)-isothiazolon und 2-Methyl-3(2H)-isothiazolon mit Magnesiumchlorid und Magnesiumnitrat, Chlorophen, 2-Chloracetamid, N,N"-Bis(4-chlorphenyl)-3,12-diimino-2,4,11,13-tetraaza-tetradecandiimidamid, sein Azetat, Gluconat und Hydrochlorid, 3-Phenoxy-1-propanol, Alkyl(C12-22)trimethylammoniumbromid und -chlorid, 4,4-Dimethyl-1,3-oxazolidin, N-Hydroxymethyl-N-[1,3-di(hydroxy-methyl)-2,5-dioxo-imidazolidinyl-4-yl]-TN-hydroxymethyl-harnstoff, Benzolcarboximidamid, 4,4'-(1,6-Hexandiylbis(oxy))bis- und seine Salze darunter Isethionat und þ-Hydroxybenzoat, Glutaraldehyd (1,5-pentandial), 5-Ethyl-3,7-dioxa-1-azabicyclo [3.3.0] octan, 3-(p-Chlorphenoxy)-1,2-propandiol, Natriumhydroxymethylaminoacetat, Silberchlorid aufgebracht auf Titandioxid, Benzolmethanaminium, N,N-Dimethyl-N-[2-[2-[4-(1,1,3,3,-tetramethylbutyl)phenoxy]ethoxy]ethyl]-, Chlorid, Benzalkoniumchlorid, -bromid und -saccharinat, Phenylmethoxy-Methanol-, 3-lod-2-propinylbutylcarbamat, 2-Methyl-2H-isothiazol-3-on,
wobei unter dem Begriff "Salze" die Salze der Kationen Natrium, Kalium, Calcium, Magnesium, Ammonium und Äthanolamine und Salze der Anionen Chlorid, Bromid, Sulfat, Azetat sowie unter dem Begriff "Ester" die Methyl-, Ethyl-, Propyl-, Isopropyl-, Butyl-, Isobutyl- und Phenylester verstanden werden.

Im Stand der Technik werden neben traditionellen Konservierungsmitteln gemäß Anhang V der VO (EG) Nr. 1223/2009 zunehmend multifunktionale Additive eingesetzt, die neben ihrer antimikrobiellen Wirkung noch über zusätzliche kosmetische, z.B. komplexbildende oder beduftende Eigenschaften verfügen. Solche multifunktionellen Stoffe können ebenfalls einen Beitrag zur biologischen Stabilisierung eines kosmetischen Produktes leisten. Erfindungsgemäße Formulierungen verzichten auf die Anwesenheit von linearen oder verzweigten, gesättigten oder ungesättigten aliphatischen Hydroxamsäuren mit 6 - 16 Kohlenstoffatomen, wie z.B. Caprylohydroxamsäure, sowie aromatischen Alkoholen der allgemeinen Struktur Ar-(CH2)n-OH mit n = 2-4.

Vorzugsweise enthält eine erfindungsgemäße Formulierung keine weiteren Konservierungsmittel bzw. Mikrobiozide.

Neben den in den Beispielformulierungen genannten Komponenten können erfindungsgemäße Formulierungen weitere Rohstoffe enthalten, wie sie üblicherweise in tensidhaltigen Formulierungen verwendet werden. Handelt es sich bei diesen Formulierungen um kosmetische Produkte zur Reinigung von Haut und/oder Haaren, so können dies beispielsweise Feuchteregulierer und Benetzmittel, zusätzliche Tenside, Perlglanzwachse, Konsistenzgeber, Verdickungsmittel, Konditionierer, Siliconverbindungen, Antischuppenmittel, Komplexbildner, Filmbildner, Quellmittel, Hydrotrope, Parfümöle, Farbstoffe etc. sein, die nachstehend exemplarisch aufgelistet sind.

### Feuchteregulierer und Benetzmittel

Als Feuchteregulierer und Benetzmittel kommen hauptsächlich lineare oder verzweigte Polyole mit einer Kettenlänge von 4 bis 12 Kohlenstoffatomen in Frage, z.B. Pentylenglycol, 1,2-Hexandiol, Heptylenglycol, Caprylyl Glycol, Hexylenglycol, oder beliebige Mischungen der linearen oder verzweigten Polyole. Auch Monoglyceride von Fettsäuren mit einer Kettenlänge von 4-12 Kohlenstoffatomen z.B. Glyceryl Caprate oder Glycerinether von linearen oder verzweigten Alkoholen mit 4-8 C-Atomen z.B. Ethylhexylglycerin kommen zu diesem Zweck in Einsatz und können allein oder in Kombination in erfindungsgemäßen Formulierungen anwesend sein.

### Tenside

Neben den erfindungsgemäß aufgeführten Tenside können als oberflächenaktive Stoffe weitere anionische, nichtionische, kationische und/oder amphotere bzw. zwitterionische Tenside enthalten sein.

Typische Beispiele für weitere anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäurethionate, Fettsäuresarcoinate, Fettsäuretauride, N-Acylaminosäuren, wie beispielsweise Acyllactylate, Acyltartrate und Acylaspartate, Alkyloligoglucosidulfate, Proteinfettsäurekondensate (insbesondere planzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettsäurepolyglycerylester, Fettalkoholpolyglycolether, Alkylphenolpolyglycolether, Fettsäurepolyglycolester, Fettsäureamidpolyglycolether, Fettaminpolyglyolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisothionate, Fettsäuresarcosinate, Fettsäuretauride, Fettsäureglutamate, α-Olefinsulfonate, Ethercarbonsäuren, Alkyloligoglucoside, Fettsäureglucamide, Amphoacetale und/oder Proteinfettsäurekondensate, letztere vorzugsweise auf Basis von Weizenproteinen.

### Perlqlanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierten Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 - 15 Kohlenstoffatomen und 2 bis 10 Hydroxylgruppen sowie deren Mischungen.

### Konsistenzgeber und Verdickungsmittel

Durch die Zugaben von 2-Methyl-1 ,3-propandiol kann es zu einer Viskositätserniedrigung der Formulierung kommen. Für einen Wiederaufbau der Viskosität können neben einer Erhöhung der Elektrolytkonzentration durch Zugabe von anorganischen Salzen auch Konsistenzgeber und Verdickungsmittel eingesetzt werden.

Als Konsistenzgeber kommen Alkyloligoglucoside allein oder in Kombination mit Fettsäuremonoglyceriden in Betracht. Weitere geeignete Verdickungsmittel können ausgewählt werden aus der Gruppe der Aerosil-Typen (Hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und diester von Fettsäuren, Polyacrylate (z. B. Carbopole^{®} und Permulene-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Weiter in Frage kommen Tenside wie beispielsweise ethoxylierte Fettsäureglyceride oder ethoxylierte Alkylglucose-Fettsäureester, von denen sich insbesondere das PEG-120 Methylglucose Dioleate (Antil 127) zur Viskositätserhöhung bewährt hat.

### Konditionierer

Kationische Polymere werden vielfach als Konditionierer in Produkten zur Reinigung und Pflege der Haut oder der Haare verwendet. Geeignete kationische Polymere sind beispielsweise kationische Cellulosederivate, wie z. B. Polyquternium-10, kationische Stärken oder deren Gemische wie sie z.B. von der Dr. Straetmans GmbH unter den Namen Amylomer und symbio^{®}quat angeboten werden, Copolymere von Diallylammoniumsalzen und Acrylamiden, quaternierte Vinylpyrrolidon/Vinilimidazol-Polymere, Kondensationsprodukte von Polyglycolen und Aminen, quaternierte Kollagenpolypeptide, wie beispielsweise Lauryldimonium Hydroxypropyl Hydrolyzed Collagen, quaternierte Weizenpolypeptide, Polyethylenimin, kationische Siliconpolymere, wie z. B. Amodimethicone, Copolymere der Adipinsäure und Dimethylaminohydroxypropyldiethylentriamin, Copolymere der Acrylsäure mit Dimethyldiallylammoniumchlorid, Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationischer Guar-Gum, sowie quaternierte Ammoniumsalz-Polymere.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/ Methylmethacrylat/ tert.-Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/ Vinylcaprolactam-Terpolymere sowie gegebenenfalls derivatisierte Celluloseether und Silicone in Frage.

### Siliconverbindungen

Gemeinsam mit quaternisierten Polymeren bilden Siliconverbindungen sogenannte Koazervate, welche zu einer Verbesserung von Kämmbarkeit und Glanz in Produkten zur Reinigung der Haare beitragen. Geeignete Siliconverbindungen sind beispielsweise Dimethylpolysiloxane, Methylphenylpolysiloxane, zyklische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen neben Lactylaten von Fettsäuren mit einer Kettenlänge von 8-14 C-Atomen auch Pirocton Olamin, Climbazole, Ketoconazol, Elubiol, Selendisulfid, Schwefel kolloidal, Schwefelpolyethylenglykolsorbitanmono-oleat, Schwefelricinolpolyethoxylat, Schwefel-Teer Destillate, Undexylensäure Monoethanolamid Sulfosuccinat Na-Salz, Zinkpyrithion, Aluminiumpyrithion und Magnesiumpyrithion / Dipyrithion-Magnesiumsulfat in Frage.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange),Wurzeln (Macis, Angelica, Sellerie, Kardamom, Costus, Iris, Calmus), Hölzern (Pinien- Sandel- Guajak-, Zedern-, Rosenholz), Kräutern und Grsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambraxon, Indol, Hedione, Sandelice, Citronenöl, Mandarinenöl, Orangenöl, Allylamylglycolat, Cyclovertal, Lavandinöl, Muskateller Salbeiöl, □-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Aus der Gruppe der organischen Säuren seien beispielhaft genannt Lävulinsäure, Zimtsäure oder Anissäure.

Als Aromen kommen beispielsweise Pfefferminzöl, Krauseminzöl, Anisöl, Sternanisöl, Kümmelöl, Eukalyptusöl, Fenchelöl, Zitronenöl, Wintergrünöl, Nelkenöl, Menthol und dergleichen in Frage.

Einige Aromen und Duftstoffe können selbst antimikrobielle Eigenschaften aufweisen oder in Kombination mit Konservierungsmitteln oder Konservierungsmittelhelfern deren antimikrobielle Wirkung synergistisch verstärken. Die synergistischen Wirkungen dieser Duftstoffe sind ausdrücklich in dieser Patentschrift inbegriffen.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I. 42051), Indigotin (C.I. 73015), Chlorophyllin (C.I. 75810), Chinolongelb (C.I. 47005), Titandioxid (C.I. 77891), Indanthrenblau RS (C.I. 69800) und Krapplack (C.I. 58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Formulierung, eingesetzt.

Gegenstand der Erfindung ist ferner die Verwendung von 2-Methyl-1,3-propandiol als alleiniges Biozid zur mikrobiologischen Stabilisierung einer wässrigen Formulierung enthaltend:
a) mindestens ein anionisches Tensid ausgewählt aus der Gruppe der Alkylsulfate, der Alkylethersulfate und/oder der Acylglutamate,
b) mindestens ein amphoteres Tensid ausgewählt aus der Gruppe der Alkylbetaine und/oder Amidoalkylbetaine.

Die Formulierung "alleiniges Biozid" umfasst, dass neben 2-Methyl-1,3-propandiol noch weitere multifunktionelle Rohstoffe in der Formulierung anwesend sein können, die neben einer weiteren Funktion ebenfalls einen Beitrag zur Inhibierung mikrobiellen Wachstums leisten können. Soweit diese nicht ausdrücklich in einen der Ansprüche ausgeschlossen werden, wird deren Gegenwart in erfindungsgemäßen Formulierungen mit beansprucht.

Die erfindungsgemäßen Formulierungen können dem Stand der Technik entsprechend hergestellt werden und weisen, wie nachfolgenden Testformulierungen belegen, eine hervorragende Stabilität gegenüber den für tensidhaltige, insbesondere kosmetisch Formulierungen relevanten und in einem mikrobiologischen Belastungstest verwendeten Keimen auf.

Relevante Keime sind insbesondere *Staphylococcus Aureus, Pseudomonas Aeruginosa, Escherichia Coli, Candida Albicans* und/oder *Aspergillus Brasiliensis.*

Die Wirksamkeit übertrifft, wie der direkte Vergleich zeigt, die Wirkung eines Konservierungssystems des Stands der Technik (Tensidbase 2b) deutlich. Nachfolgende Beispiele sollen die vorliegende Erfindung verdeutlichen, ohne diese einzuschränken.

### Vergleichsbeispiel 1

Eine O/W-Emulsion wurde hergestellt, die der technischen Lehre der FR 2780283 A1 entspricht, jedoch nicht den Komplexbildner Na4 EDTA enthält (alle Angaben sind Gewichtsteile):

| O/W-Emulsion 1 , pH = 6,5 | | | |
|---|---|---|---|
| Phase | Rohstoff | INCI (EU) | % |
| A | Deionised Water | Aqua | 66.80 |
| | dermofeel^{®} PA-3 | Sodium Phytate; Aqua; Alcohol | 0.10 |
| | MP-Diol Glycol | Methylpropandiol | 5,00 |
| | Glycerin 99.5% | Glycerin | 5.00 |
| A1 | Keltrol CG-RD | Xanthan Gum | 0.30 |
| B | dermofeel^{®} GSC | Glyceryl Stearate Citrate | 3.50 |
| | Miglyol 812 N | Caprylic/Capric Triglyceride | 6.00 |
| | Phytosqualan, veg. grade | Squalane | 6.00 |
| | Sunflower Oil | Helianthus Annuus Seed Oil | 5.00 |
| | Lanette O | Cetearyl Alcohol | 2.00 |
| | Perf. Nat. Sunny Pomegranate P0250284 | Parfum | 0.30 |
| | | | 100.0 |

Ein Konservierungsmittelbelastungstest gemäß Pharm. Eur. 2014, 5.1.3 zeigte:

| O/W-Emulsion 1, pH = 6,5 | 0 Tage | 2 Tage | 7 Tage | 14 Tage | 28 Tage |
|---|---|---|---|---|---|
| Staphylococcus Aureus | 3,9 × 10⁵ | 1,6 × 10⁵ | 2,1 × 10⁵ | 2,0 × 10⁵ | 1,3 × 10⁵ |
| Pseudomonas Aeruginosa | 5,8 × 10⁵ | 1,1 × 10⁵ | 8,0 × 10⁴ | 6,2 × 10⁴ | 1,2 × 10⁴ |
| Escherichia Coli | 6,1 × 10⁵ | 8,0 × 10⁴ | 1,2 × 10⁵ | 1,1 × 10⁵ | 2,3 × 10⁴ |
| Candida Albicans | 3,4 × 10⁵ | 2,2 × 10⁵ | 2,9 × 10⁵ | 3,6 × 10⁵ | 5,5 × 10⁵ |
| Aspergillus Brasiliensis | 3,2 × 10⁵ | 1,0 × 10⁵ | 8,4 × 10⁴ | 6,1 × 10⁴ | 8,0 × 10⁴ |

Man erkennt, dass ein Einsatz von 2-Methyl-1,3-propandiol nicht automatisch und zwangsläufig die gewünschte mikrobiozide Wirkung zeigt, sondern dass die erfindungsgemäß beanspruchte Kombination wesentlich für den Erfolg der Erfindung ist.

### Vergleichsbeispiel 2 und Beispiele 1 bis 3

Die in der nachfolgenden Tabelle genannten Formulierungen wurden hergestellt. Tensidbase 2b ist Vergleichsbeispiel 2, Tensidbase 1, 2a und 3 sind die erfindungsgemäßen Beispiele 1 bis 3. Die Prozentangaben sind Gewichtsteile.

| Rohstoff | INCI | Tensidbase 1 | Tensidbase 2a | Tensidbase 2b | Tensidbase 3 |
|---|---|---|---|---|---|
| Texapon N 70 | Sodium Laureth Sulfate; Aqua | 12,0 % | 10,0 % | 10,0 % | - |
| Tego Betain F 50 | Cocoamidopropyl Betaine; Aqua | 8,0 % | 7,0 % | 7,0 % | 9,0 % |
| Am isoft CS-22 | Disodium Cocoyl Glutamate; Sodium Cocoyl Glutamate; Aqua | - | 5,0 % | 5,0 % | 5,0 % |
| Plantacare 1200 UP | Lauryl Glucoside; Aqua | - | 5,0% | 5,0% | 13,5 % |
| Lamesoft PO 65 | Coco Glucoside, Glyceryl Oleate | - | - | - | 2,0% |
| MP-Diol Glycol | Methylpropandiol | 5,0 % | 3,0 % | | 5,0% |
| Verstatil PC | Phenoxyethanol, Caprylyl Glycol | - | - | 1,0 % | - |
| Water | Aqua | Ad 100 % | Ad 100 % | | Ad 100 % |
| pH | | 6,5 | 7,5 | 7,0 | 6,5 |

Sämtliche Tests wurden entsprechend der Vorgaben für einen Konservierungsmittelbelastungstest gemäß Pharm. Eur. 2014, 5.1.3 durchgeführt.

| Tensidbase 1 | 0 Tage | 2 Tage | 7 Tage | 14 Tage | 28 Tage |
|---|---|---|---|---|---|
| Staphylococcus Aureus | 3,9 × 10⁵ | < 10 | < 10 | < 10 | < 10 |
| Pseudomonas Aeruginosa | 5,1 × 10⁵ | < 10 | < 10 | < 10 | < 10 |
| Escherichia Coli | 3,7 × 10⁵ | < 10 | < 10 | < 10 | < 10 |
| Candida Albicans | 7,0 × 10⁵ | < 10 | < 10 | < 10 | < 10 |
| Aspergillus Brasiliensis | 3,6 × 10⁵ | 7,0 × 10⁴ | 1,7 × 10⁴ | < 10 | < 10 |

| Tensidbase 2a | 0 Tage | 2 Tage | 7 Tage | 14 Tage | 28 Tage |
|---|---|---|---|---|---|
| Staphylococcus Aureus | 3,9 × 10⁵ | < 10 | < 10 | < 10 | < 10 |
| Pseudomonas Aeruginosa | 5,1 × 10⁵ | < 10 | < 10 | < 10 | < 10 |
| Escherichia Coli | 3,7 × 10⁵ | < 10 | < 10 | < 10 | < 10 |
| Candida Albicans | 7,0 × 10⁵ | < 10 | < 10 | < 10 | < 10 |
| Aspergillus Brasiliensis | 3,6 × 10⁵ | 4,8 × 10⁴ | 10 | < 10 | < 10 |

| Tensidbase 2b | 0 Tage | 2 Tage | 7 Tage | 14 Tage | 28 Tage |
|---|---|---|---|---|---|
| Staphylococcus Aureus | 6,3 × 10⁵ | 8,0 × 10⁴ | 3,7 × 10⁴ | 2,2 × 10⁴ | 4,0 × 10³ |
| Pseudomonas Aeruginosa | 7,0 × 10⁵ | 1,2 × 10⁵ | 1,2 × 10⁵ | 1,2 × 10⁵ | 1,0 × 10⁵ |
| Escherichia Coli | 9,0 × 10⁵ | 8,0 × 10⁴ | 1,0 × 10⁵ | 1,0 × 10⁵ | 1,0 × 10⁵ |
| Candida Albicans | 7,2 × 10⁵ | 4,0 × 10⁴ | 6,0 × 10⁴ | 6,0 × 10⁴ | 1,8 × 10⁴ |
| Aspergillus Brasiliensis | 3,0 × 10⁵ | 6,0 × 10⁴ | 6,4 × 10⁴ | 6,4 × 10⁴ | 4,0 × 10⁴ |

| Tensidbase 3 | 0 Tage | 2 Tage | 7 Tage | 14 Tage | 28 Tage |
|---|---|---|---|---|---|
| Staphylococcus Aureus | 3,9 × 10⁵ | < 10 | < 10 | < 10 | < 10 |
| Pseudomonas Aeruginosa | 5,1 × 10⁵ | < 10 | < 10 | < 10 | < 10 |
| Escherichia Coli | 3,7 × 10⁵ | < 10 | < 10 | < 10 | < 10 |
| Candida Albicans | 7,0 × 10⁵ | < 10 | < 10 | < 10 | < 10 |
| Aspergillus Brasiliensis | 3,6 × 10⁵ | 4,6 × 10⁴ | < 10 | < 10 | < 10 |

Die Tests zeigen die Überlegenheit der erfindungsgemäßen Formulierungen.

## Patentansprüche

1. Wässrige Formulierung enthaltend:
a) mindestens ein anionisches Tensid ausgewählt aus der Gruppe der Alkylsulfate, der Alkylethersulfate und/oder der Acylglutamate,
b) mindestens ein amphoteres Tensid ausgewählt aus der Gruppe der Alkylbetaine und/oder Amidoalkylbetaine,
c) 2-Methyl-1,3-propandiol,
**dadurch gekennzeichnet, dass**
d) sie einen pH Wert von 6 bis 10 aufweist,
e) zur mikrobiologischen Stabilisierung keine weiteren Konservierungsmittel enthält ausgewählt aus der Gruppe bestehend aus:
e1) im Anhang V der VO (EG) Nr. 1223/2009 genannten, zugelassenen Konservierungsmittel ausgewählt aus Benzoesäure und ihr Natriumsalz und andere Salze der Benzoesäure und Benzoesäureester, Propionsäure und ihre Salze, Salicylsäure und ihre Salze, 2,4-Hexadiensäure und ihre Salze, Formaldehyd und Paraformaldehyd, 2-Hydroxybiphenyl und seine Salze, Pyrithionzink, Anorganische Sulfite und Bisulfite , Chlorobutanol, 4-Hydroxybenzoesäure, ihre Salze und Ester, 3-Acetyl-6-methyl-2,4(3H)-pyrandion und seine Salze, Ameisensäure und ihr Natriumsalz, 1,6-Bis(4-amidino-2-bromphenoxy)-n-hexan (Dibromhexamidin) und seine Salze einschließlich Isethionat, Thiomersal, Phenylquecksilber und seine Salze einschließlich Borat, 10-Undecylensäure und seine Salze, 5-Pyrimidinamin, 1,3-Bis(2-ethylhexyl)hexahydro-5-methyl, 5-Brom-5-nitro-1,3-dioxan, Bronopol, 2,4-Dichlorbenzylalkohol, 1-(4-Chlorphenyl)-3-(3,4-dichlorphenyl)-harnstoff, Chlorkresol, 5-Chloro-2-(2,4-dichlorophenoxy)-phenol, Chloroxylenol, N,N"-Methylenbis[N'-[3-(hydroxymethyl)-2,5-dioxoimidazolidin-4-yl]harnstoff, Poly(methylen), α, ω-Bis[[[(aminoiminomethyl)amino]iminomethyl]amino]-, Dihydrochlorid, 2-Phenoxyethanol, Methenamin, 1-(3-Chloroallyl)-3,5,7-triaza-1-azonia- adamantanchlorid, 1-(4-Chlorphenoxy)1-(1H-imidazol-1-yl)-3,3-dimethyl-2-butanon, 1,3-Bis-(hydroxy-methyl)-5,5-dimethyl-2,4-imidazolidindion, Benzylalkohol, 1-Hydroxy-4-methyl-6-(2,4,4-trimethyl-pentyl)-2-pyridon und sein Monoethanolaminsalz, 2,2'-Methylenbis(6-brom-4-chlorphenol) (Bromchlorophen), 3-Methyl-4-(1-methyläthyl)phenol, Gemisch von 5-Chlor-2-methyl-3(2H)-isothiazolon und 2-Methyl-3(2H)-isothiazolon mit Magnesiumchlorid und Magnesiumnitrat, Chlorophen, 2-Chloracetamid, N,N"-Bis(4-chlorphenyl)-3,12-diimino-2,4,11,13-tetraaza-tetradecandiimidamid, sein Azetat, Gluconat und Hydrochlorid, 3-Phenoxy-1-propanol, Alkyl(C12-22)trimethylammoniumbromid und -chlorid, 4,4-Dimethyl-1,3-oxazolidin, N-Hydroxymethyl-N-[1,3-di(hydroxy-methyl)-2,5-dioxo-imidazolidinyl-4-yl]-TN-hydroxymethyl-harnstoff, Benzolcarboximidamid, 4,4'-(1,6-Hexandiylbis(oxy))bis- und seine Salze darunter Isethionat und þ-Hydroxybenzoat, Glutaraldehyd (1,5-pentandial), 5-Ethyl-3,7-dioxa-1-azabicyclo [3.3.0] octan, 3-(p-Chlorphenoxy)-1,2-propandiol, Natriumhydroxymethylaminoacetat, Silberchlorid aufgebracht auf Titandioxid, Benzolmethanaminium, N,N-Dimethyl-N-[2-[2-[4-(1,1,3,3,-tetramethylbutyl)phenoxy]ethoxy]ethyl]-, Chlorid, Benzalkoniumchlorid, -bromid und - saccharinat, Phenylmethoxy-Methanol-, 3-lod-2-propinylbutylcarbamat, 2-Methyl-2H-isothiazol-3-on,
wobei unter dem Begriff "Salze" die Salze der Kationen Natrium, Kalium, Calcium, Magnesium, Ammonium und Äthanolamine und Salze der Anionen Chlorid, Bromid, Sulfat, Azetat sowie unter dem Begriff "Ester" die Methyl-, Ethyl-, Propyl-, Isopropyl-, Butyl-, Isobutyl- und Phenylester verstanden werden, und
e2) multifunktionalen Additiven ausgewählt aus der Gruppe der linearen oder verzweigten, gesättigten oder ungesättigten aliphatischen oder aromatischen Hydroxamsäuren mit einer Anzahl von 6 - 16 Kohlenstoffatomen und aromatischen Alkoholen der allgemeinen Struktur
Ar-(CH2)n-OH
mit n = 2 - 4,
**dadurch gekennzeichnet, dass** 2-Methyl-1,3-propandiol in einer Konzentration von 0,1 bis 8 Gew.-% enthalten ist,
wobei die folgende Zusammensetzung ausgenommen ist:
| INCI | Gew.-% |
|---|---|
| Aqua/water/eau | 86,2225 |
| Sodium laureth sulfate | 3,99 |
| Coco-betaine | 3,90 |
| Methylpropanediol | 2,00 |
| Sodium chloride | 0,91 |
| Pentylene glycol | 0,55 |
| Disodium edta | 0,50 |
| Fragrance (parfum) | 0,50 |
| Sodium citrate | 0,50 |
| Peg-90 glyceryl isostearate | 0,35 |
| Niacinamide | 0,30 |
| Citric acid | 0,14 |
| Camellia sinensis leaf extract | 0,05 |
| Tambourissa trichophylla leaf extract | 0,05 |
| Laureth-2 | 0,0375 |

2. Formulierung nach Anspruch 1, **dadurch gekennzeichnet, dass** das anionische Tensid gemäß a) in einer Konzentration von 0,1 bis 30, vorzugsweise 2 bis 25, weiter vorzugsweise 5 bis 20 Gew.-% enthalten ist.

3. Formulierung nach Anspruch 2, **dadurch gekennzeichnet, dass** das anionische Tensid gemäß a) ausgewählt ist aus der Gruppe bestehend aus Sodium Lauryl Sulfate, Ammonium Lauryl Sulfate; Sodium Laureth Sulfate, Sodium Cocoyl Glutamate und Disodium Cocoyl Glutamate.

4. Formulierung nach einem der Ansprüche 1 bis 3, **dadurch gekennzeichnet, dass** das amphotere Tensid gemäß b) in einer Konzentration 0,1 bis 20, vorzugsweise 1 bis 15, weiter vorzugsweise 2 bis 10 Gew.-% enthalten ist.

5. Formulierung nach Anspruch 4, **dadurch gekennzeichnet, dass** das amphotere Tensid gemäß b) ein quaternäres Tensid ausgewählt aus der Gruppe bestehend aus Cocoamidopropyl Betain und Coco Betaine ist.

6. Formulierung nach einem der Ansprüche 1 bis 5, **dadurch gekennzeichnet, dass** 2-Methyl-1,3-propandiol in einer Konzentration von 1 bis 8, weiter vorzugsweise 2 bis 7 Gew.-% enthalten ist.

7. Formulierung nach einem der Ansprüche 1 bis 6, **dadurch gekennzeichnet, dass** der pH-Wert 6 bis 9,5, vorzugsweise 6 bis 9 beträgt.

8. Kosmetisches Produkt zur Reinigung und/oder Pflege der Haut und/oder Haare, **dadurch gekennzeichnet, dass** es eine Formulierung nach einem der Ansprüche 1 bis 6 enthält.

9. Nicht-therapeutische Verwendung einer Formulierung nach einem der Ansprüche 1 bis 6 in einem kosmetischen Produkt zur Reinigung und/oder Pflege der Haut und/oder Haare.

10. Verwendung von 2-Methyl-1,3-propandiol als alleiniges Biozid zur mikrobiologischen Stabilisierung einer wässrigen Formulierung enthaltend:
a) mindestens ein anionisches Tensid ausgewählt aus der Gruppe der Alkylsulfate, der Alkylethersulfate und/oder der Acylglutamate,
b) mindestens ein amphoteres Tensid ausgewählt aus der Gruppe der Alkylbetaine und/oder Amidoalkylbetaine.

11. Verwendung nach Anspruch 9 zur mikrobiologischen Stabilisierung der Formulierung gegen *Staphylococcus Aureus, Pseudomonas Aeruginosa, Escherichia Coli, Candida Albicans* und/oder *Aspergillus Brasiliensis.*

12. Verwendung nach Anspruch 9 oder 10, **dadurch gekennzeichnet, dass** 2-Methyl-1,3-propandiol in einer Konzentration von 0,1 bis 10, vorzugsweise 1 bis 8, weiter vorzugsweise 2 bis 7 Gew.-% eingesetzt wird.

## Claims

1. Aqueous formulation comprising:
a) at least one anionic surfactant selected from the group of alkyl sulfates, alkyl ether sulfates and/or acyl glutamates,
b) at least one amphoteric surfactant selected from the group of alkyl betaines and/or amidoalkyl betaines,
c) 2-methyl-1,3-propanediol,
**characterized in that**
d) said formulation has a pH of 6 to 10,
e) comprises no further preservatives for microbiological stabilization selected from the group consisting of:
e1) authorized preservatives cited in Annex V of Regulation (EC) No. 1223/2009 selected from benzoic acid and sodium salt thereof and other salts of benzoic acid and benzoic esters, propionic acid and salts thereof, salicylic acid and salts thereof, 2,4-hexadienoic acid and salts thereof, formaldehyde and paraformaldehyde, 2-hydroxybiphenyl and salts thereof, zinc pyrithione, inorganic sulfites and bisulfites, chlorobutanol, 4-hydroxybenzoic acid, salts and esters thereof, 3-acetyl-6-methyl-2,4(3H)-pyrandione and salts thereof, formic acid and sodium salt thereof, 1,6-bis(4-amidino-2-bromophenoxy)-n-hexane (dibromohexamidine) and salts thereof including isethionate, thiomersal, phenylmercury and salts thereof including borate, 10-undecylenic acid and salts thereof, 5-pyrimidinamine, 1,3-bis(2-ethylhexyl)hexahydro-5-methyl, 5-bromo-5-nitro-1,3-dioxane, bronopol, 2,4-dichlorobenzyl alcohol, 1-(4-chlorophenyl)-3-(3,4-dichlorophenyl)urea, chlorocresol, 5-chloro-2-(2,4-dichlorophenoxy)phenol, chloroxylenol, N,N"-methylenebis[N'-[3-(hydroxymethyl)-2,5-dioxoimidazolidin-4-yl]urea, poly(methylene), α,ω-bis[[[(aminoiminomethyl)amino]iminomethyl]amino]-, dihydrochloride, 2-phenoxyethanol, methenamine, 1-(3-chloroallyl)-3,5,7-triaza-1-azoniaadamantane chloride, 1-(4-chlorophenoxy)-1-(1H-imidazol-1-yl)-3,3-dimethyl-2-butanone, 1,3-bis(hydroxymethyl)-5,5-dimethyl-2,4-imidazolidinedione, benzyl alcohol, 1-hydroxy-4-methyl-6-(2,4,4-trimethylpentyl)-2-pyridone and monoethanolamine salt thereof, 2,2'-methylenebis(6-bromo-4-chlorophenol) (bromochlorophen), 3-methyl-4-(1-methylethyl) phenol, mixture of 5-chloro-2-methyl-3(2H)-isothiazolone and 2-methyl-3(2H)-isothiazolone with magnesium chloride and magnesium nitrate, chlorophene, 2-chloroacetamide, N,N"-bis(4-chlorophenyl)-3,12-diimino-2,4,11,13-tetraazatetradecanediimidamide, acetate, gluconate and hydrochloride thereof, 3-phenoxy-1-propanol, alkyl(C12-22)trimethylammonium bromide and chloride, 4,4-dimethyl-1,3-oxazolidine, N-hydroxymethyl-N-[1,3-di(hydroxymethyl)-2,5-dioxoimidazolidinyl-4-yl]-TN-hydroxymethylurea, benzolcarboximidamide, 4,4'-(1,6-hexanediylbis(oxy))bis- and salts thereof including isethionate and þ-hydroxybenzoate, glutaraldehyde (1,5-pentanedial), 5-ethyl-3,7-dioxa-1-azabicyclo [3.3.0] octane, 3-(p-chlorophenoxy)-1,2-propanediol, sodium hydroxymethylamino acetate, silver chloride applied to titanium dioxide, benzenemethanaminium, N,N-dimethyl-N-[2-[2-[4-(1,1,3,3,-tetramethylbutyl)phenoxy]ethoxy]ethyl]-, chloride, benzalkonium chloride, benzalkonium bromide and benzalkonium saccharinate, phenylmethoxymethanol, 3-iodo-2-propynyl butylcarbamate, 2-methyl-2H-isothiazol-3-one,
wherein the term "salts" is understood to mean the salts of the cations sodium, potassium, calcium, magnesium, ammonium and ethanolamine and salts of the anions chloride, bromide, sulfate, acetate and the term "esters" is understood to mean methyl, ethyl, propyl, isopropyl, butyl, isobutyl and phenyl esters, and
e2) multifunctional additives selected from the group of linear or branched, saturated or unsaturated aliphatic or aromatic hydroxamic acids having a number of 6 - 16 carbon atoms and aromatic alcohols of the general structure
Ar-(CH2)n-OH
where n = 2 - 4,
**characterized in that** 2-methyl-1,3-propanediol is present at a concentration of 0.1 to 8% by weight,
wherein the following composition is excluded:
| INCI | wt% |
|---|---|
| Aqua/water/eau | 86.2225 |
| Sodium laureth sulfate | 3.99 |
| Coco-betaine | 3.90 |
| Methylpropanediol | 2.00 |
| Sodium chloride | 0.91 |
| | |
|---|---|
| Pentylene glycol | 0.55 |
| Disodium EDTA | 0.50 |
| Fragrance (perfume) | 0.50 |
| Sodium citrate | 0.50 |
| PEG-90 glyceryl isostearate | 0.35 |
| Niacinamide | 0.30 |
| Citric acid | 0.14 |
| Camelia sinensis leaf extract | 0.05 |
| Tambourissa trichophylla leaf extract | 0.05 |
| Laureth-2 | 0.0375 |

2. Formulation according to Claim 1, **characterized in that** the anionic surfactant according to a) is present at a concentration of 0.1 to 30, preferably 2 to 25, more preferably 5 to 20% by weight.

3. Formulation according to Claim 2, **characterized in that** the anionic surfactant according to a) is selected from the group consisting of sodium lauryl sulfate, ammonium lauryl sulfate; sodium laureth sulfate, sodium cocoyl glutamate and disodium cocoyl glutamate.

4. Formulation according to any of Claims 1 to 3, **characterized in that** the amphoteric surfactant according to b) is present at a concentration of 0.1 to 20, preferably 1 to 15, more preferably 2 to 10% by weight.

5. Formulation according to Claim 4, **characterized in that** the amphoteric surfactant according to b) is a quaternary surfactant selected from the group consisting of cocoamidopropyl betaine and coco betaine.

6. Formulation according to any of Claims 1 to 5, **characterized in that** 2-methyl-1,3-propanediol is present at a concentration of 1 to 8, more preferably 2 to 7% by weight.

7. Formulation according to any of Claims 1 to 6, **characterized in that** the pH is from 6 to 9.5, preferably 6 to 9.

8. Cosmetic product for cleansing and/or care of skin and/or hair, **characterized in that** said product comprises a formulation according to any of Claims 1 to 6.

9. Non-therapeutic use of a formulation according to any of Claims 1 to 6 in a cosmetic product for cleansing and/or care of skin and/or hair.

10. Use of 2-methyl-1,3-propanediol as sole biocide for microbiological stabilization of an aqueous formulation comprising:
a) at least one anionic surfactant selected from the group of alkyl sulfates, alkyl ether sulfates and/or acyl glutamates,
b) at least one amphoteric surfactant selected from the group of alkyl betaines and/or amidoalkyl betaines.

11. Use according to Claim 9 for microbiological stabilization of the formulation against *Staphylococcus aureus, Pseudomonas aeruginosa, Escherichia coli, Candida albicans* and/or *Aspergillus brasiliensis.*

12. Use according to Claim 9 or 10, **characterized in that** 2-methyl-1,3-propanediol is used at a concentration of 0.1 to 10, preferably 1 to 8, more preferably 2 to 7% by weight.

## Revendications

1. Formulation aqueuse contenant :
a) au moins un tensioactif anionique choisi dans le groupe des sulfates d'alkyle, des sulfates d'alkyléther et/ou des glutamates d'acyle,
b) au moins un tensioactif amphotère choisi dans le groupe des alkylbétaïnes et/ou des amidoalkylbétaïnes,
c) du 2-méthyl-1,3-propanediol,
**caractérisée en ce que**
d) elle présente une valeur de pH de 6 à 10,
e) pour la stabilisation microbiologique, elle ne contient pas d'autres agents conservateurs choisis dans le groupe constitué par :
e1) les agents conservateurs autorisés, mentionnés dans l'annexe V du règlement (CE) n° 1223/2009, choisis parmi l'acide benzoïque et son sel de sodium et d'autres sels de l'acide benzoïque et les esters de l'acide benzoïque, l'acide propionique et ses sels, l'acide salicylique et ses sels, l'acide 2,4-hexadiénoïque et ses sels, le formaldéhyde et le paraformaldéhyde, le 2-hydroxybiphényle et ses sels, le pyrithione de zinc, les sulfites et bisulfites inorganiques, le chlorobutanol, l'acide 4-hydroxybenzoïque, ses sels et esters, la 3-acétyl-6-méthyl-2,4(3H)-pyranedione et ses sels, l'acide formique et son sel de sodium, le 1,6-bis(4-amidino-2-bromophénoxy)-n-hexane (dibromohexamidine) et ses sels, y compris l'iséthionate, le thiomersal, le phénylmercure et ses sels, y compris le borate, l'acide 10-undécylénique et ses sels, la 1,3-bis(2-éthylhexyl)hexahydro-5-méthyl-5-pyrimidinamine, le 5-bromo-5-nitro-1,3-dioxane, le bronopol, l'alcool 2,4-dichlorobenzylique, la 1-(4-chlorophényl)-3-(3,4-dichlorophényl)-urée, le chlorocrésol, le 5-chloro-2-(2,4-dichlorophénoxy)-phénol, le chloroxylénol, la N,N"-méthylènebis[N'-[3-(hydroxyméthyl)-2,5-dioxoimidazolidin-4-yl]urée, le dichlorhydrate d'α,ω-bis[[[(aminoiminométhyl)amino]-iminométhyl]amino]poly(méthylène), le dichlorhydrate, le 2-phénoxyéthanol, la méthénamine, le chlorure de 1-(3-chloroallyl)-3,5,7-triaza-1-azonia-adamantane, la 1-(4-chlorophénoxy)1-(1H-imidazol-1-yl)-3,3-diméthyl-2-butanone, la 1,3-bis-(hydroxy-méthyl)-5,5-diméthyl-2,4-imidazolidinedione, l'alcool benzylique, la 1-hydroxy-4-méthyl-6-(2,4,4-triméthylpentyl)-2-pyridone et son sel de monoéthanolamine, le 2,2'-méthylènebis(6-bromo-4-chlorophénol) (bromochlorophène), le 3-méthyl-4-(1-méthyléthyl)phénol, le mélange de 5-chloro-2-méthyl-3(2H)-isothiazolone et de 2-méthyl-3(2H)-isothiazolone avec du chlorure de magnésium et du nitrate de magnésium, le chlorophène, le 2-chloroacétamide, le N,N"-bis(4-chlorophényl)-3,12-diimino-2,4,11,13-tétraaza-tétradécanediimidamide, son acétate, gluconate et chlorhydrate, le 3-phénoxy-1-propanol, le bromure et le chlorure d'alkyl(C12-22)triméthylammonium, la 4,4-diméthyl-1,3-oxazolidine, la N-hydroxyméthyl-N-[1,3-di(hydroxy-méthyl)-2,5-dioxo-imidazolidinyl-4-yl]-TN-hydroxyméthyl-urée, le 4,4'-(1,6-hexanediylbis(oxy))bisbenzènecarboximidamide et ses sels, incluant l'iséthionate et le p-hydroxybenzoate, le glutaraldéhyde (1,5-pentanedial), le 5-éthyl-3,7-dioxa-1-azabicyclo[3.3.0]octane, le 3-(p-chlorophénoxy)-1,2-propanediol, l'hydroxyméthylaminoacétate de sodium, le chlorure d'argent déposé sur du dioxyde de titane, le chlorure de N,N-diméthyl-N-[2-[2-[4-(1,1,3,3,-tétraméthylbutyl)phénoxyl]éthoxy]éthyl]-benzèneméthanaminium, le chlorure, le bromure et le saccharinate de benzalconium, le phénylméthoxy-méthanol, le carbamate de 3-iodo-2-propynylbutyle, la 2-méthyl-2H-isothiazol-3-one,
le concept "sels" désignant les sels des cations sodium, potassium, calcium, magnésium, ammonium et éthanolamine et les sels des anions chlorure, bromure, sulfate, acétate et le concept "esters" désignant les esters de méthyle, d'éthyle, de propyle, d'isopropyle, de butyle, d'isobutyle et de phényle et
e2) les additifs multifonctionnels choisis dans le groupe des acides hydroxamiques linéaires ou ramifiés, saturés ou insaturés, aliphatiques ou aromatiques comprenant un nombre de 6 à 16 atomes de carbone et des alcools aromatiques de structure générale
Ar-(CH₂)ₙ-OH
dans laquelle n = 2 à 4,
**caractérisée en ce que** le 2-méthyl-1,3-propanediol est contenu en une concentration de 0,1 à 8 % en poids,
la composition suivante étant exclue :
| INCI | % en poids |
|---|---|
| Eau | 86,2225 |
| Laurethsulfate de sodium | 3, 99 |
| Bétaïne de coco | 3, 90 |
| Méthylpropanediol | 2,00 |
| Chlorure de sodium | 0,91 |
| Pentylèneglycol | 0,55 |
| EDTA disodique | 0,50 |
| Parfum | 0,50 |
| Citrate de sodium | 0,50 |
| Isostéarate de PEG-90 glycéryle | 0,35 |
| Niacinamide | 0,30 |
| Acide citrique | 0,14 |
| Extrait de feuilles de Camellia sinensis | 0,05 |
| Extrait de feuilles de Tambourissa trichophylla | 0,05 |
| Laureth-2 | 0,0375 |

2. Formulation selon la revendication 1, **caractérisée en ce que** le tensioactif anionique selon a) est contenu en une concentration de 0,1 à 30, de préférence de 2 à 25, plus préférablement de 5 à 20% en poids.

3. Formulation selon la revendication 2, **caractérisée en ce que** le tensioactif anionique selon a) est choisi dans le groupe constitué par le laurylsulfate de sodium, le laurylsulfate d'ammonium, le laurethsulfate de sodium, le cocoylglutamate de sodium et le cocoylglutamate disodique.

4. Formulation selon l'une quelconque des revendications 1 à 3, **caractérisée, en ce que** le tensioactif amphotère selon b) est contenu en une concentration de 0,1 à 20, de préférence de 1 à 15, plus préférablement de 2 à 10 % en poids.

5. Formulation selon la revendication 4, **caractérisée en ce que** le tensioactif amphotère selon b) est un tensioactif quaternaire choisi dans le groupe constitué par la cocoamidopropylbétaïne et la cocobétaïne.

6. Formulation selon l'une quelconque des revendications 1 à 5, **caractérisée en ce que** le 2-méthyl-1,3-propanediol est contenu en une concentration de 1 à 8, plus préférablement de 2 à 7 % en poids.

7. Formulation selon l'une quelconque des revendications 1 à 6, **caractérisée en ce que** le pH est de 6 à 9,5, de préférence 6 à 9.

8. Produit cosmétique pour le nettoyage et/ou le soin de la peau et/ou des cheveux, **caractérisé en ce qu'**il contient une formulation selon l'une quelconque des revendications 1 à 6.

9. Utilisation non thérapeutique d'une formulation selon l'une quelconque des revendications 1 à 6 dans un produit cosmétique pour le nettoyage et/ou le soin de la peau et/ou des cheveux.

10. Utilisation de 2-méthyl-1,3-propanediol comme unique biocide pour la stabilisation microbiologique d'une formulation contenant :
a) au moins un tensioactif anionique choisi dans le groupe des sulfates d'alkyle, des sulfates d'alkyléther et/ou des glutamates d'acyle,
b) au moins un tensioactif amphotère choisi dans le groupe des alkylbétaïnes et/ou des amidoalkylbétaïnes.

11. Utilisation selon la revendication 9 pour la stabilisation microbiologique de la formulation contre Staphylococcus aureus, Pseudomonas aeruginosa, Escherichia Coli, Candida albicans et/ou Aspergillus brasiliensis.

12. Utilisation selon la revendication 9 ou 10, **caractérisée en ce que** le 2-méthyl-1,3-propanediol est utilisé en une concentration de 0,1 à 10, de préférence de 1 à 8, plus préférablement de 2 à 7 % en poids.
